# EUROPÄISCHE PATENTSCHRIFT

(11) **EP 1 424 041 B1**
(45) Veröffentlichungstag und Bekanntmachung des Hinweises auf die Patenterteilung: **03.08.2005**
(21) Anmeldenummer: 02025942.0
(22) Anmeldetag: 20.11.2002
(51) Int. Cl.: A61B 17/132

(54) **Venenstauvorrichtung**
Vein occluding device
Dispositif d'occlusion veineuse

(43) Veröffentlichungstag der Anmeldung: 02.06.2004
(73) Patentinhaber: PRÄMETA GmbH & Co. KG, 51107 Köln (DE)
(72) Erfinder: Heisig, Rolf, Dr., 72768 Reutlingen (DE); Pyschik, Roman, 42859 Remscheid (DE)
(74) Vertreter: Dallmeyer, Georg, Dipl.-Ing.

(56) Entgegenhaltungen:
- EP-A- 0 374 464
- EP-A- 1 078 603
- DE-A- 3 431 728
- DE-A- 3 624 112
- GB-A- 2 138 490

## Beschreibung

Die Erfindung bezieht sich auf eine Venenstauvorrichtung für Körperteile, mit einem eine Schlaufe bildenden Band, mit einem Schlossgehäuse und einem in dem Schlossgehäuse schwenkbar angeordneten Klemmhebel zum Klemmen des durch das Schlossgehäuse hindurchgeführten Bandabschnittes mit einem ersten Ende des Klemmhebels bei gespannter Schlaufe, wobei ein Ende des Bandes an dem zweiten Ende des Klemmhebels angreift und der Klemmhebel mit Hilfe einer Rastverbindung in dem Schlossgehäuse einrastbar ist und mittels einer am Klemmhebel angeordneten Tastfläche gegen die Wirkung einer Feder von dem Schlossgehäuse lösbar ist.

Die bekannten Venenstauvorrichtungen bzw. Abschnürrvorrichtung für Körperteile sind üblicherweise so ausgebildet, dass zur schwenkbaren Lagerung des Klemmhebels ein Achsstift vorgesehen ist, der beiderseits in den Seitenwangen des Schlossgehäuses gelagert ist. Der Klemmhebel ist daher fest in dem Schlossgehäuse integriert. Das eine Ende des Bandes ist durch das Schlossgehäuse hindurchgeführt und wird in diesem bei um das Körperteil gespannter Schlaufe mittels des Klemmhebels in seiner Position innerhalb des Schlossgehäuses festgehalten. Das andere Ende des Bandes ist mittels eines Halteteils an dem der Klemmstelle gegenüberliegenden Ende des Klemmhebels einrastbar. Bei Druckausübung auf den Klemmhebel kann das gespannte Band gelöst werden. Wird Druck auf das Halteorgan ausgeübt, kann das Bandende von dem Klemmhebel gelöst werden.

Eine derartige Venenstauvorrichtung ist beispielsweise aus der EP-A-0 374 464 bekannt. Nachteilig an der bekannten Konstruktion einer Venenstauvorrichtung ist die Vielzahl der erforderlichen Einzelteile, sowie der Montageaufwand zur Herstellung der Venenstauvorrichtung.

Aus der EP 1078603 ist desweiteren ein gattungsgemäßer Venenstauer bekannt. Bei dieser Venenstauvorrichtung sind zum Entspannen der Schlaufe und zum vollständigen Lösen des Bandes von dem Schlossgehäuse zwei unterschiedliche Tasten vorgesehen. Dafür dass für das Entspannen der Schlaufe und für das Lösen des Klemmhebels von dem Schlossgehäuse unterschiedliche Bedienelemente vorhanden sind, kann die Reihenfolge der Bedienung der Tastflächen verwechselt werden, so dass die Gefahr eines Stauschocks besteht.

Auch bei Venenstauvorrichtungen, die durch Niederdrücken des Klemmhebels sowohl das Entspannen der Schlaufe als auch Lösen des Klemmhebels von dem Schlossgehäuse ermöglichen, besteht die Gefahr, dass die voneinander separat vorzunehmenden Schritte, Entstauen und Lösen, zu schnell erfolgen, so dass auch in diesem Fall die Gefahr des Stauschocks nur unzureichend beseitigt ist. Eine derartige Venenstauvorrichtung ist beispielsweise in der DE-A-3624112 beschrieben.

Der Erfindung liegt die Aufgabe zugrunde, eine Venenstauvorrichtung der eingangs genannten Art derart zu verbessern, dass Herstellung, Montage und Bedienung vereinfacht werden und dabei gleichzeitig die Bedienungssicherheit erhöht wird.

Zur Lösung dieser Aufgabe dienen die Merkmale des Anspruchs 1.

Die Erfindung sieht in vorteilhafter Weise vor, dass bei Betätigung des Klemmhebels in Abhängigkeit von der Hubbewegung der Tastfläche zum Entspannen der Schlaufe und zum vollständigen Lösen des Klemmhebels von dem Schlossgehäuse eine unterschiedliche Federkraft entgegen der Bewegungsrichtung der Tastfläche wirkt.

Die Erfindung stellt in vorteilhafter Weise sicher, dass zwangsweise zuerst die vorgespannte Schlaufe entspannt und erst dann der Klemmhebel von dem Schlossgehäuse gelöst werden kann. Dadurch, dass die Feder für beide Funktionen der Tastfläche des Klemmhebels eine unterschiedliche Federkraft entgegen der Bewegungsrichtung der Tastfläche ausübt, sind die Funktionen, Entstauen und Lösen, für eine Bedienungsperson bei der Betätigung der Tastfläche eindeutig unterscheidbar.

Es ist vorgesehen, dass die Federkraft in Abhängigkeit des von der Hubbewegung der Tastfläche sich verändernden Federweges zweistufig abgestuft ist.

Dabei kann in Abhängigkeit von der Hubbewegung der Tastfläche eine unterschiedlich große Kontaktfläche der Feder mit dem Klemmhebel im Eingriff sein. In Abhängigkeit der Größe der Kontaktfläche ergibt sich eine unterschiedliche Federkennung der Feder, so dass sich der Widerstand bei Betätigung der Tastfläche verändert.

Der Klemmhebel weist an der Unterseite des ersten Endes eine zu diesem Ende ansteigende Einführungsfläche auf. Diese Einführungsfläche ermöglicht ein leichtes Einführen des Klemmhebels in das Schlossgehäuse.

An der Unterseite des Klemmhebels unterhalb der Rastverbindung befindet sich eine in Richtung zum ersten Ende und zur Rastverbindung aufsteigende Schrägfläche. Diese Schrägfläche erzeugt beim Einführen des Klemmhebels in das Schlossgehäuse eine fühlbare Widerstandskraft, so dass das spätere Einrasten der Rastverbindung eindeutig wahrgenommen werden kann. Beim Lösen des Klemmhebels von dem Schlossgehäuse bewirkt diese Schrägfläche eine Unterstützung der Auswurfbewegung.

Die Schrägfläche kann in einer Aussparung des Klemmhebels oder an einem Vorsprung des Klemmhebels ausgebildet sein.

Desweiteren kann der Klemmhebel an der Unterseite eine mit der Feder zusammenwirkenden Andruckfläche aufweisen, die die Arbeitslage des Klemmhebels im Gehäuse sichert, wenn ein Rastvorsprung der Rastverbindung in einer Rastaussparung des Schlossgehäuses sitzt.

Die Lage der Feder zwischen der Rastverbindung und dem der Schlaufe zugewandten Ende des Schlossgehäuses ist dabei für die Funktion der Schrägfläche und der Andruckfläche wesentlich. Durch die Lage der Feder an dem der Schlaufe zugewandten Ende des Schlossgehäuses kann die Feder ein wirksames Gegenmoment auf den Klemmhebel ausüben, wenn dieser durch Druck auf die Tastfläche niedergedrückt wird.

Die Feder erstreckt sich vorzugsweise quer über den durch das Schlossgehäuse hindurchgeführten Wandabschnitt.

Der Klemmhebel ist in dem Schlossgehäuse schwenkbar, ohne selbst eine in dem Schlossgehäuse gelagerte Achse aufzuweisen.

Der wesentliche Vorteil der Erfindung besteht darin, dass aufgrund eines Druckpunktes bei der Druckausübung auf die Tastfläche für die Bedienungsperson offensichtlich ist, wann die Funktion Entspannen der Schlaufe, endet und ab wann das Lösen des Klemmhebels von dem Schlossgehäuse beginnt, so dass ganz sicher die Gefahr eines Stauschocks vermieden wird. Die Funktionen der Tastfläche des Klemmhebels sind somit eindeutig getrennt, so dass es nicht auf das Geschick der Bedienungsperson ankommt, die auf die Tastfläche ausgeübte Kraft exakt zu dosieren.

Im folgenden werden unter Bezugnahme auf die Zeichnungen Ausführungsbeispiele der Erfindung näher erläutert.

Es zeigen:
- Fig. 1: die erfindungsgemäße Venenstauvorrichtung,
- Fig. 2: einen Längsschnitt durch den Klemmhebel und die Feder,
- Fig. 3: einen Querschnitt durch die Venenstauvorrichtung in Höhe der Rastverbindung,
- Fig. 4a: die Belastung der Feder zum Entspannen der Schlaufe,
- Fig. 4b: die Belastung der Feder beim vollständigen Lösen des Klemmhebels von dem Schlossgehäuse,
- Fign. 5a bis 5e: unterschiedliche Ausführungsformen einer quer angeordneten Feder, und
- Fig. 6a bis 6d: unterschiedliche Federquerschnittsprofile in Verbindung mit unter schiedlichen Schrägflächen des Klemmhebels.

Die Venenstauvorrichtung 1 als Abschnürvorrichtung für Körperteile weist ein Schlossgehäuse 4 auf, in dem ein Klemmhebel 6 schwenkbar angeordnet ist. Der Klemmhebel 6 dient zum Klemmen eines Bandabschnittes 3 eines eine Schlaufe bildenden Bandes 2. Ein Ende 8 des Bandes ist an dem hinteren Ende 10 des Klemmhebels 6 befestigt, wobei das andere Ende des Bandes durch das Schlossgehäuse 4 hindurchgeführt ist. Ein Bandabschnitt 3 des Bandes 2 wird in dem Schlossgehäuse 4 durch den Klemmhebel 6 festgeklemmt, sobald die Schlaufe des Bandes 2 unter Spannung steht. Hierzu wird das Band 2 um das abzuschnürende Körperteil gelegt, wobei der Klemmhebel 6 vorher oder nachträglich in das Schlossgehäuse 4 eingeführt und dort mit Hilfe einer Rastverbindung 12 verrastet wird. Anschließend wird an dem freien Bandende gezogen bis die Schlaufe des Bandes 2 das Körperteil eng umfaßt und unter Spannung steht. Steht die Schlaufe 2 unter Spannung, wird der Klemmhebel 6 so verschwenkt, dass er mit dem vorderen Ende 7 eine Klemmstelle für den Bandabschnitt 3 in dem Schlossgehäuse 4 bildet, so dass eine Selbsthemmung des Bandes 2 eintritt.

Der Klemmhebel 6 ist nicht durch eine Achse in dem Schlossgehäuse 4 gelagert, sondern wird schwenkbar in seiner eingerasteten Position durch die Rastverbindung 12 und eine unter dem Klemmhebel 6 angeordneten Feder 20 gehalten, die gegen eine Andrückfläche 30 auf der Unterseite des Klemmhebels 6 anliegt.

Der Klemmhebel 6 ist in dem Schlossgehäuse 4 einrastbar, wobei die Rastverbindung 12 aus einem Vorsprung 11 an dem oberen Schenkelteil 22 und einer Aussparung 13 in dem oberen Teil 5 des Schlossgehäuses 4 besteht. Der Klemmhebel 6 ist von dem Schlossgehäuse 4 durch Betätigung einer Tastfläche 18 am hinteren Ende des Klemmhebels 6 nach vorheriger Entspannung des Bandes lösbar. Bei erhöhter Druckausübung auf diese Tastfläche 18 über einen voreingestellten Druckpunkt hinaus wird der Vorsprung 11 außer Eingriff mit der Aussparung 13 gebracht, so dass der Klemmhebel 6 vollständig aus dem Schlossgehäuse 4 entnehmbar ist.

Bis zum Erreichen des Druckpunktes wird der Klemmhebel 6 nur so weit verschwenkt, dass ein langsames Entspannen der Schlaufe erfolgen kann, so dass kein Stauschock entstehen kann.

Bei Betätigung der Tastfläche 18 gegen die Wirkung der Feder 20 ist demzufolge ein unterschiedlicher Druck erforderlich, um die gemeinsamen Funktionen der Tastfläche 18, nämlich das Entspannen der Schlaufe und das vollständige Lösen des Klemmhebels 6 von dem Schlossgehäuse 4, voneinander zu trennen. Vorzugsweise weist hierzu die Feder 20 eine von dem Federweg abhängige unterschiedliche Federkennung auf. Dies kann bei bestimmten Ausführungsbeispielen durch unterschiedliche Abschnitte der Feder 20 realisiert werden oder durch ein unterschiedlichen Eingriff des Klemmhebels 6 mit der Feder 20.

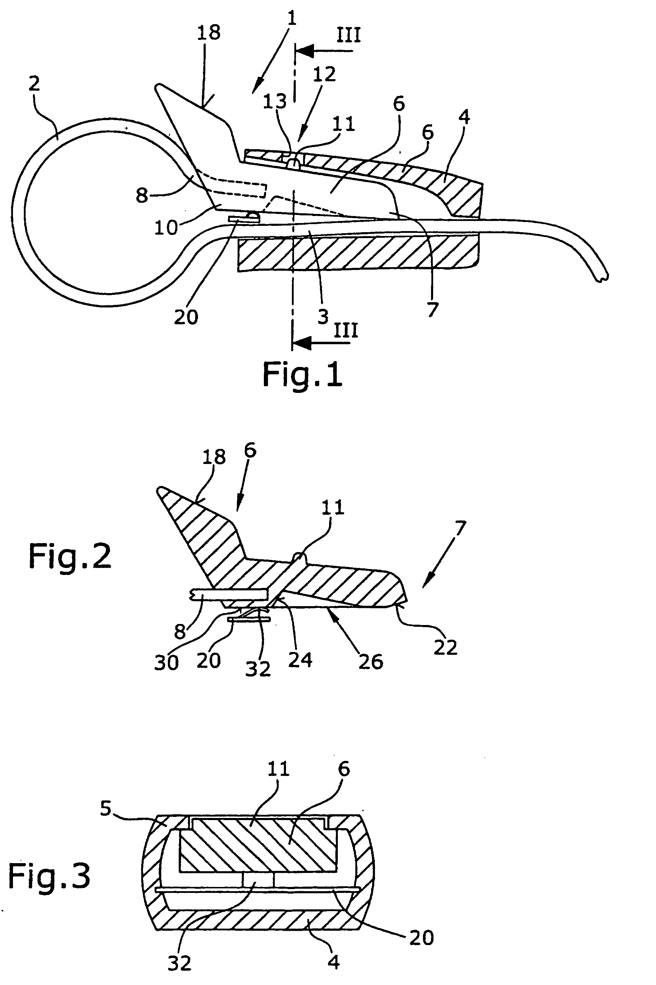

Fig. 2 zeigt einen Schnitt durch die Längsmittelebene des Klemmhebels 6.

Die Anordnung der Feder 20 ist hinter einer Schrägfläche 24 und zwar in Richtung auf die von dem Band 2 gebildeten Schlaufe gesehen, zwischen der Rastverbindung 12 und dem der Schlaufe zugewandten Ende des Schlossgehäuses 4 angeordnet.

Die Feder 20 wirkt mit einer Andruckfläche 30 auf der Unterseite des Klemmhebels 6 zusammen, wobei die Feder 20 den Klemmhebel 6 um einen Schwenkpunkt an dem vorderen Ende 7 des Klemmhebels 6 nach oben gegen das Schlossgehäuse vorspannt. Die Fign. 1 und 2 zeigen unterschiedliche sich quererstreckende Federn 20, die beispielsweise in den Seitenwangen des Schlossgehäuses 4 gelagert sind und sich quer zu dem Bandabschnitt 3 erstrecken.

An dem vorderen Ende 7 des Klemmhebels 6 kann wie in Fig. 2 gezeigt eine Einführungsfläche 22 vorgesehen, die das Einführen des Klemmhebels 6 in das Schlossgehäuse 4 erleichtert. Die bereits erwähnte Schrägfläche 24 erzeugt dadurch, dass die Feder 20 beim Einführen des Klemmhebels 6 gegen diese Schrägfläche anliegt, eine spürbare Gegenkraft, so dass der Klemmhebel 6 spürbar nach Überwindung dieses Widerstandes in die Rastverbindung 12 einrastet.

Andererseits unterstützt die Schrägfläche 24 bei Betätigung der Tastenfläche 18 zum vollständigen Lösen des Klemmhebels 6 von dem Schlossgehäuse 4 den Auswurf des Klemmhebels 6 indem die Feder 20 hinter der Schrägfläche 24 eingreift und Druck auf die Schrägfläche 24 ausübt.

Fig. 3 zeigt ein Querschnitt durch das Schlossgehäuse mit eingesetztem Klemmhebel 6.

Das Band 2 ist in Fig. 3 nicht gezeigt. Die Feder 20 weist einen nach oben abstehenden Federteil 32 auf, der im Eingriff mit dem Klemmhebel 6 ist.

Es kann vorgesehen sein, dass der vorstehende Federteil 32 eine weichere Federkennung aufweist, so dass bei Betätigung des Klemmhebels 6 zunächst der vorstehende Federteil 32 verformt wird, um ein Entspannen der gespannten Schlaufe zu ermöglichen. Anschließend bei Erhöhung des Druckes auf die Tastfläche 18 wird die gesamte Feder 20 verformt, wobei die auf den Klemmhebel 6 wirkende Feder erheblich höher ist, so dass zum Lösen des Klemmhebels 6 von dem Schlossgehäuse 4 ein Druckpunkt nach vorgegebener Hubbewegung der Tastfläche 18 überwunden werden muss, um den Klemmhebel 6 von dem Schlossgehäuse 4 zu lösen.

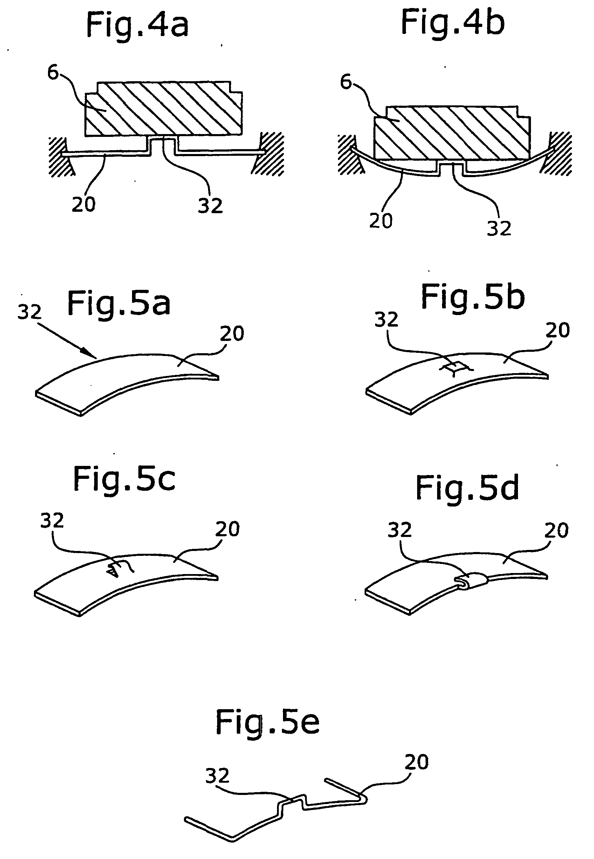

Die Fign. 4a und 4b veranschaulichen eine alternative Lösungsmöglichkeit, bei der der Vorsprung 32 nicht unbedingt eine geringere Federsteifigkeit aufweisen muss. In diesem Fall wird die erhöhte Widerstandskraft der Feder dadurch erzielt, dass zunächst nur ein Teil, nämlich der vorstehende Federabschnitt 32 im Eingriff mit dem Klemmhebel 6 ist und ein Druckpunkt dadurch entsteht, dass gemäß Fig. 4b die Kontaktfläche zwischen dem Klemmhebel 6 und der Feder 20 vergrößert wird, wodurch eine erheblich höhere Kraft erforderlich ist, um den Klemmhebel 6 weiter niederzudrücken und die Rastverbindung 12 zu lösen.

Die Fign. 5a bis 5e zeigen alternative Ausführungsbeispiele einer Feder 20, die nach oben gewölbt ist. Auch bei dem Ausführungsbeispiel der Fig. 5a wird ein Druckpunkt erzeugt, indem die Kontaktflächen zwischen dem Klemmhebel 6, ähnlich wie in Fig. 4b gezeigt, vergrößert wird.

Bei den Ausführungsbeispielen der Fign. 5c bis 5e besteht ausdrücklich die Möglichkeit, die Federkennung des vorstehenden Federabschnittes 32 weicher zu gestalten, so dass eine Feder 20 mit einer vom Federweg abhängigen stufenförmigen Veränderung der Federkennung geschaffen wird.

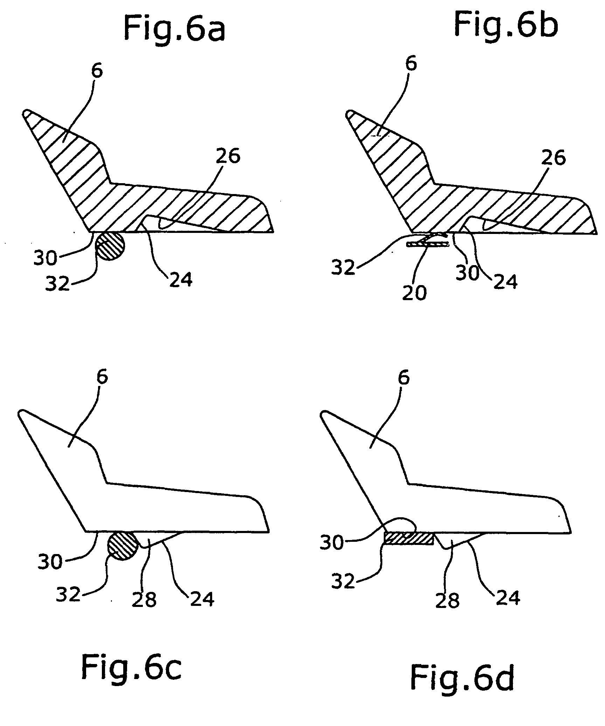

Die Fign. 6a bis 6d zeigen unterschiedliche Ausführungsbeispiele der Erfindung, bei denen die Schrägfläche 24 entweder in einer Aussparung 26 angeordnet ist oder (Fign. 6c und 6d) alternativ an einem nach unten abstehenden Nocken 28 des Klemmhebels 6 vorgesehen ist.

Desweiteren sind in den Fign. 6a bis 6d unterschiedliche Querschnittsprofile der sich quererstreckenden Feder 20 dargestellt.

## Patentansprüche

1. Venenstauvorrichtung (1) für Körperteile, mit einem eine Schlaufe bildenden Band (2), mit einem Schlossgehäuse (4) und einem in dem Schlossgehäuse (4) schwenkbar angeordneten Klemmhebel (6) zum Klemmen des durch das Schlossgehäuse (4) hindurchgeführten Bandabschnittes (3) mit einem ersten Ende (7) des Klemmhebels (6) bei gespannter Schlaufe, wobei ein Ende (8) des Bandes (2) an dem zweiten Ende (10) des Klemmhebels (6) angreift, wobei der Klemmhebel (6) mit Hilfe einer Rastverbindung (12) in das Schlossgehäuse (4) einrastbar ist und mittels einer am Klemmhebel (6) angeordneten Tastfläche (18) gegen die Wirkung einer Feder (20) von dem Schlossgehäuse (4) lösbar ist,
wobei
bei Betätigung des Klemmhebels (6) in Abhängigkeit von der Hubbewegung der Tastfläche (18) zur Unterscheidung der Funktionen Entspannen der Schlaufe und vollständiges Lösen des Klemmhebels (6) von dem Schlossgehäuse (4) eine unterschiedliche Federkraft entgegen der Bewegungsrichtung der Tastfläche (18) wirkt, **dadurch gekennzeichnet, dass**
die Federkraft in Abhängigkeit des von der Hubbewegung der Tastfläche (18) sich verändernden Federweges zweistufig abgestuft ist.

2. Venenstauer nach Anspruch 1, **dadurch gekennzeichnet, dass** in Abhängigkeit von der Hubbewegung der Tastfläche (18) eine unterschiedlich große Kontaktfläche der Feder (20) mit dem Klemmhebel (6) im Eingriff ist.

3. Venenstauer nach Anspruch 1 oder 2, **dadurch gekennzeichnet, dass** der Klemmhebel (6) an der Unterseite des ersten Endes (7) eine zum Ende (7) ansteigende Einführungsfläche (22) aufweist.

4. Venenstauer nach einem der Ansprüche 1 bis 3, **dadurch gekennzeichnet, dass** an der Unterseite des Klemmhebels (6) unterhalb der Rastverbindung (12) eine in Richtung zum ersten Ende (7) und zur Rastverbindung (12) aufsteigende Schrägfläche (24) angeordnet ist.

5. Venenstauer nach Anspruch 4, **dadurch gekennzeichnet, dass** die Schrägfläche (24) in einer Aussparung (26) oder an einem Vorsprung (28) ausgebildet ist.

6. Venenstauer nach einem der Ansprüche 1 bis 5, **dadurch gekennzeichnet, dass** der Klemmhebel (6) an der Unterseite eine mit der Feder (20) zusammenwirkende Andruckfläche (30) aufweist.

7. Venenstauer nach Anspruche 6, **dadurch gekennzeichnet, dass** ein in Richtung auf den Klemmhebel (6) vorstehender Abschnitt (32) der Feder (20) mit der Andruckfläche (30) in Kontakt ist.

8. Venenstauvorrichtung nach einem der Ansprüche 1 bis 7, **dadurch gekennzeichnet, dass** die Feder (20) sich quer über dem Bandabschnitt (3) erstreckt.

9. Venenstauvorrichtung nach einem der Ansprüche 1 bis 8, **dadurch gekennzeichnet, dass** die Feder (20) an dem der Schlaufe zugewandten Ende (10) des Schlossgehäuses (4) in Richtung der Schlaufe hinter der Rastverbindung (12) angeordnet ist.

## Claims

1. Tourniquet device (1) for extremities comprising a strap (2) forming a loop, with a lock housing (4) and a clamping lever (6) pivotally arranged in the lock housing (4) for clamping the strap portion (3) passed through the lock housing (4) by a first end (7) of the clamping lever (6) when the loop is taut, one end (8) of the strap (2) engaging a second end (10) of the clamping lever (6), which clamping lever (6) may be caught in the lock housing (4) by means of a catch connection (12) and may be detached from the lock housing (4) against the action of a spring (20) by means of a key surface (18) provided on the clamping lever (6), wherein, depending on the stroke of the key surface (18), a different spring force acts against the direction of movement of the key surface (18) upon actuation of the clamping lever (6) to differentiate between the functions of loosening the loop and of a complete detachment of the clamping lever (6) from the lock housing (4),
**characterized in that**
the spring force is two-staged in dependences on the spring path changing as a function of the stroke of the key surface (18).

2. Tourniquet device of claim 1, wherein, depending on the stroke of the key surface (18), a differently sized contact surface of the spring (20) is in engagement with the clamping lever (6).

3. Tourniquet device of claim 1 or 2, wherein the lower side of the first end (7) of the clamping lever (6) has an insertion surface (22) rising towards the end (7).

4. Tourniquet device of one of claims 1 to 3, wherein an inclined surface (24) rising towards the first end (7) and the catch connection (12) is provided on the lower side of the clamping lever (6) below the catch connection (12).

5. Tourniquet device of claim 4, wherein the inclined surface (24) is formed in a recess (26) or at a projection (28).

6. Tourniquet device of one of claims 1 to 5, wherein the lower side of th clamping lever (6) has a pressure surface (30) cooperating with the spring (20).

7. Tourniquet device of claim 6, wherein a portion (32) of the spring (20) projecting towards the clamping lever (6) is in contact with the pressure surface (30).

8. Tourniquet device of one of claims 1 to 7, wherein the spring (20) extends across the strap portion (3).

9. Tourniquet device of one of claims 1 to 8, wherein the spring (20) is arranged at the end (10) of the lock housing (4) facing the loop and, seen in the direction of the loop, behind the catch connection (12).

## Revendications

1. Dispositif d'occlusion veineuse (1) pour des parties du corps, comportant une bande (2) formant une boucle, un boîtier de serrure (4) et un levier de serrage (6) placé de manière à pouvoir pivoter dans le boîtier de serrure (4) pour serrer la portion de bande (3) introduite dans le boîtier de serrure (4) avec une première extrémité (7) du levier de serrage (6) lorsque la boucle est tendue, une extrémité (8) de la bande (2) étant en prise avec la deuxième extrémité (10) du levier de serrage (6), le levier de serrage (6) pouvant s'encliqueter dans le boîtier de serrure (4) à l'aide d'une liaison par encliquetage (12) et pouvant être libéré du boîtier de serrure (4), à l'encontre de l'action d'un ressort (20), et au moyen d'une surface de touche (18) située sur le levier de serrage (6), une force de ressort différente opposée au sens de déplacement de la surface de touche (18) agissant en fonction de la course de la surface de touche (18), lorsque l'on actionne le levier de serrage (6), afin de distinguer les fonctions de desserrage de la boucle et de libération totale du levier de serrage (6) du boîtier de serrure (4), **caractérisé en ce que** la force du ressort présente deux niveaux en fonction de la course du ressort se modifiant par la course de la surface de touche (18).

2. Dispositif d'occlusion veineuse selon la revendication 1, **caractérisé en ce qu'**une surface de contact d'importance variable du ressort (20) est en prise avec le levier de serrage (6) en fonction de la course de la surface de touche (18).

3. Dispositif d'occlusion veineuse selon la revendication 1 ou 2, **caractérisé en ce que** le levier de serrage (6) comporte du côté inférieur de la première extrémité (7) une face d'insertion (22) montant en direction de l'extrémité (7).

4. Dispositif d'occlusion veineuse selon l'une des revendications 1 à 3, **caractérisé en ce qu'**une face oblique (24), s'élevant en direction de la première extrémité (7) et pour la liaison par encliquetage (12), est située du côté inférieur du levier de serrage (6), au-dessous de la liaison par encliquetage (12).

5. Dispositif d'occlusion veineuse selon la revendication 4, **caractérisé en ce que** la face oblique (24) est formée dans un évidement (26) ou sur une saillie (28).

6. Dispositif d'occlusion veineuse selon l'une des revendications 1 à 5, **caractérisé en ce que** le levier de serrage (6) comporte du côté inférieur une face de pressage (30) coopérant avec le ressort (20).

7. Dispositif d'occlusion veineuse selon la revendication 6, **caractérisé en ce qu'**une portion (32) du ressort (20), qui est saillante en direction du levier de serrage (6), est en contact avec la face de pressage (30).

8. Dispositif d'occlusion veineuse selon l'une des revendications 1 à 7, **caractérisé en ce que** le ressort (20) s'étend transversalement au-dessus de la portion de bande (3).

9. Dispositif d'occlusion veineuse selon l'une des revendications 1 à 8, **caractérisé en ce que** le ressort (20) est placé à l'extrémité (10), dirigée vers la boucle, du boîtier de serrure (4), en arrière de la liaison par encliquetage (12) en direction de la boucle.
